(19) Europäisches Patentamt
European Patent Office
Office européen des brevets

(11) Veröffentlichungsnummer : **0 049 891**
**B1**

(12) **EUROPÄISCHE PATENTSCHRIFT**

(45) Veröffentlichungstag der Patentschrift : **30.01.85**

(51) Int. Cl.[4] : **C 07 D499/04**, C 07 D499/78// C07D295/18

(21) Anmeldenummer : **81108244.5**

(22) Anmeldetag : **12.10.81**

(54) **Verfahren zur Herstellung von 6-Aminopenicillansäurederivaten und Zwischenprodukte dafür.**

(30) Priorität : **15.10.80 CH 7700/80**

(43) Veröffentlichungstag der Anmeldung : **21.04.82 Patentblatt 82/16**

(45) Bekanntmachung des Hinweises auf die Patenterteilung : **30.01.85 Patentblatt 85/05**

(84) Benannte Vertragsstaaten : **AT CH IT LI LU NL SE**

(56) Entgegenhaltungen :
**CH-A- 581 141**
**CHEMICAL ABSTRACTS, Band 86, Nr. 25, 20. Juni 1977, Seite 515, Nr. 188771e Columbus, Ohio, U.S.A. J.O. DZIEGIELEWSKI et al.: "Gamma-radiolysis of carboxybenzyl-penicillin. Effect of a benzyl group substituent on radiolysis of solid penicillin"**
**TETRAHEDRON LETTERS, Nr. 3, 1967, Seiten 215-217, Pergamon Press Ltd. G.B. A.P. KRAPCHO et al.: "The decarbethoxylation of geminal dicarbethoxy compounds"**

(73) Patentinhaber : **F. HOFFMANN-LA ROCHE & CO. Aktiengesellschaft**
**CH-4002 Basel (CH)**

(72) Erfinder : **Furlenmeier, André, Dr.**
**Wettsteinallee 119**
**CH-4058 Basel (CH)**

(74) Vertreter : **Lederer, Franz, Dr. et al**
**Patentanwälte Dr. Lederer Franz Meyer-Roxlau Reiner F. Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 049 891 B1

**Beschreibung**

Die vorliegende Erfindung betrifft ein neues Verfahren zur Herstellung von 6-Aminopenicillansäurederivaten der allgemeinen Formel

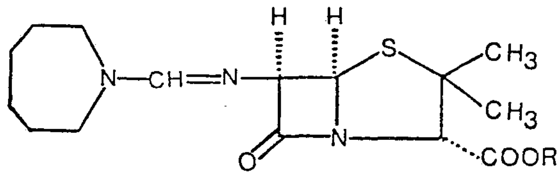

(I)

in der R Wasserstoff oder eine leicht hydrolysierbare Estergruppe darstellt,
sowie von pharmazeutisch verträglichen Salzen dieser Verbindungen, welches Verfahren dadurch gekennzeichnet ist, dass man eine Verbindung der allgemeinen Formel

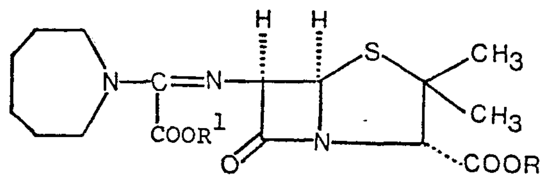

(II)

in der R die oben gegebene Bedeutung hat, und $R^1$ eine hydrogenolytisch abspaltbare Gruppe, wie Benzhydryl oder insbesondere Benzyl, bedeutet,
oder ein Salz einer solchen Verbindung in Gegenwart von Palladium/Kohle hydriert, wonach man erwünschtenfalls einen erhaltenen Ester der Formel I einer Esterspaltung unterwirft, erwünschtenfalls eine erhaltene Carbonsäure der Formel I mit einem die Estergruppe R abgebenden Mittel behandelt und erwünschtenfalls eine erhaltene Verbindung der Formel I in ein pharmazeutisch verträgliches Salz davon überführt.

Die erfindungsgemässe katalytische Hydrierung der Ausgangsverbindungen der Formel II bewirkt, wie gefunden wurde, nicht nur die Abspaltung der Gruppe $R^1$ sondern auch eine Decarboxylierung, sodass im Endeffekt die Gruppe $—COOR^1$ durch ein H ersetzt wird.

Die erfindungsgemässe katalytische Hydrierung erfolgt in Gegenwart von Palladium/Kohle. Die Hydrierung wird vorzugsweise bei atmosphärischem Druck in einem polaren Lösungsmittel durchgeführt, z. B. in einem niederen Alkanol, z. B. Methanol, Aethanol oder Isopropanol, und in einem Temperaturbereich von etwa 0 bis 80 °C, vorzugsweise bei Raumtemperatur.

Die Verfahrensprodukte der Formel I sowie deren pharmazeutisch verträgliche Salze sind antibakteriell stark wirksam, insbesondere gegen gramnegative Bakterien und zeigen eine besonders niedrige Toxizität. Sie sind deshalb besonders geeignet für die Behandlung und Prophylaxe von Infektionskrankheiten.

Beispiele von leicht hydrolysierbaren Estergruppen R sind : Niederes Alkanoyloxyalkyl, z. B. Acetoxymethyl, Pivaloyloxymethyl, 1-Acetoxyäthyl und 1-Pivaloyloxyäthyl ; niederes Alkoxycarbonyloxyalkyl, z. B. Methoxycarbonyloxymethyl, 1-Aethoxycarbonyloxyäthyl und 1-Isopropoxycarbonyloxyäthyl ; Lactonylreste, z. B. Phthalidyl und Thiophthalidyl ; niederes Alkoxymethyl, z. B. Methoxymethyl ; und niederes Alkanoylaminomethyl, z. B. Acetamidomethyl.

Die neuen Ausgangsverbindungen der Formel II können erhalten werden durch Umsetzung von Hexamethylenimin $[(CH_2)_6NH]$ mit einem Oxalsäure-monobenzylester-halogenid $[Hal—CO—COOCH_2C_6H_5]$, vorzugsweise dem Chlorid, Behandeln des erhaltenen Oxalsäure-monobenzylester-hexamethylenimids $[(CH_2)_6N—CO—COOCH_2C_6H_5]$ mit Phosphorpentasulfid und Reaktion des gebildeten Hexamethylenimino-thioxo-essigsäurebenzylesters $[(CH_2)_6N—CS—COOCH_2C_6H_5]$ mit 6-Aminopenicillansäure oder einem leicht hydrolysierbaren Ester davon in Gegenwart eines Alkylierungsmittels, wie z. B. Triäthyloxonium-tetrafluorborat, vorzugsweise in Gegenwart eines säurebindenden Mittels, wie Triäthylamin oder Diisopropyläthylamin, und in einem inerten organischen Lösungsmittel, wie Methylenchlorid, und bei einer Temperatur im Bereiche von etwa − 10 °C und + 30 °C. Wahlweise kann eine so erhaltene Base der Formel II durch Säurezusatz, z. B. Salzsäure oder p-Toluolsulfonsäure, in das entsprechende Säureadditionssalz bzw. eine so erhaltene Carbonsäure der Formel II durch Basenzusatz, z. B. Natriumcarbonat oder Kalilauge, in das entsprechende Salz übergeführt werden.

Die nachstehenden Beispiele erläutern die Erfindung. Alle Temperaturen sind in Grad Celsius angegeben.

## Beispiel

Pivaloyloxymethyl 6-[(hexahydro-1H-azepin-1-yl)]-methylenamino-penicillinat.

22,5 g (0,03 M) Methylen-(2S, 5R, 6R)-6-[[[(benzyloxy)-carbonyl]-(hexahydro-1H-azepin-1-yl)methylen]amino]-7-oxo-4-thia-1-azabicyclo[3.2.0]heptan-2-carboxylat-pivalat-p-toluolsulfonat werden in 250 ml Aethylacetat mit überschüssiger Natriumbicarbonat-Lösung unter Eiszusatz in die freie Base übergeführt. Die Aethylacetat-Lösung wird mit Wasser gewaschen, über Magnesiumsulfat getrocknet und im Vakuum bei 25° eingedampft. Der Rückstand wird in 300 ml Isopropanol mit Palladium/Kohle (5 %) hydriert, wobei der Katalysator einmal erneuert wird. Der Katalysator wird abgesaugt, und das Filtrat bei 30° im Vakuum eingedampft, wobei Kristallisation erfolgt. Die Substanz wird aus Aceton/Wasser umkristallisiert. Smp. 117-119°. $[\alpha]_D^{25} = + 230°$ (c = 1 in Aethanol). Das NMR-Spektrum stimmt mit der Struktur überein.

Das als Ausgangsverbindung verwendete Methylen-(2S, 5R, 6R)-6-[[[(benzyloxy)carbonyl]-(hexahydro-1H-azepin-1-yl)-methylen]amino]-7-oxo-4-thia-1-azabicyclo[3.2.0]heptan-2-carboxylat-pivalat-p-toluolsulfonat kann wie folgt hergestellt werden :

Zu einer 0° kalten Lösung von 19,8 g (0,2 M) Hexamethylenimin in 70 ml Chloroform tropft man unter Rühren innerhalb 45 Minuten eine Lösung von 19,9 g (0,1 M) Oxalsäure-monobenzylesterchlorid in 70 ml Chloroform und rührt anschliessend eine Stunde bei 20°. Die Lösung wird je zweimal mit 3N wässriger Salzsäure, Wasser, 5 % wässriger Natriumbicarbonat-Lösung und Wasser gewaschen, über Magnesiumsulfat getrocknet und im Vakuum eingedampft. Das zurückbleibende Oel wird destilliert. Man erhält Oxalsäure-monobenzylester-hexamethylenimid vom Siedepunkt 165-170° (0,3 mm Torr) ; $n_D^{25} = 1{,}532\,8$.

52,2 g (0,2 M) Oxalsäure-monobenzylester-hexamethylenimid in 520 ml abs. Toluol werden nach Zusatz von 26,6 g (0,12 M) Phosphorpentasulfid 5 Stunden unter Rühren und Ausschluss von Feuchtigkeit auf 75° erwärmt. Die abgekühlte Suspension wird im Vakuum filtriert, das Filtrat im Vakuum eingedampft, das zurückbleibende Oel in Aethylacetat mit wässriger Natriumbicarbonat-Lösung, Wasser, 1N wässriger Salzsäure und Wasser gewaschen und nach dem Trocknen über Magnesiumsulfat im Vakuum eingedampft. Das zurückbleibende Oel wird in Benzol gelöst und über Kieselgel mit Benzol chromatographiert. Die den Hexamethylenimino-thioxo-essigsäurebenzylester enthaltenden Fraktionen werden gesammelt, im Vakuum eingedampft und bei 40° im Hochvakuum getrocknet. Das IR-Spektrum des erhaltenen Hexamethylenimino-thioxo-essigsäurebenzylesters stimmt mit der Struktur überein. Das nicht umgesetzte Ausgangsmaterial kann bei der Chromatographie regeneriert und erneut mit Phosphorpentasulfid umgesetzt werden.

13,9 g (0,05 M) Hexamethylenimino-thioxo-essigsäure-benzylester werden in 300 ml abs. Methylenchlorid gelöst und mit 9,5 g (0,05 M) Triäthyloxonium-tetrafluorborat versetzt und unter einem schwachen Stickstoffstrom über Nacht unter Calciumchloridverschluss stehen gelassen. Zu dieser zuvor auf 0° abgekühlten Lösung tropft man innerhalb 10 Minuten eine 0° kalte Lösung von 16,5 g (0,05 M) 6-Aminopenicillansäure-pivaloyloxymethylester und 8,6 ml (0,05 M) Diisopropyläthylamin und rührt anschliessend während 5 Stunden unter Stickstoffbegasung bei 20°. Die Lösung wird im Vakuum bei 25° eingedampft, das Oel in Aethylacetat mit wässriger Natriumbicarbonat-Lösung und wässriger 5 % Natriumchlorid-Lösung gewaschen, über Magnesiumsulfat getrocknet und im Vakuum bei 25° eingedampft. Das zurückbleibende Oel wird in 200 ml Aether gelöst und mit einer Lösung von 9 g p-Toluolsulfonsäurehydrat in 100 ml Aethylacetat versetzt und unter Rühren kristallisiert. Man erhält Methylen-(2S, 5R, 6R)-6-[[[(benzyloxy)carbonyl]-(hexahydro-1H-azepin-1-yl)methylen]-amino]-7-oxo-4-thia-1-azabicyclo[3.2.0]heptan-2-carboxylatpivalat-p-toluolsulfonat vom Schmelzpunkt 124-126° (Zers.). $[\alpha]_D^{25} = 129{,}5°$ (c = 1 in Dimethylformamid). Das NMR-Spektrum stimmt mit der Struktur überein.

**Ansprüche** (für die Vertragsstaaten : CH, IT, LI, LU, NL, SE)

1. Verfahren zur Herstellung von 6-Aminopenicillansäurederivaten der allgemeinen Formel

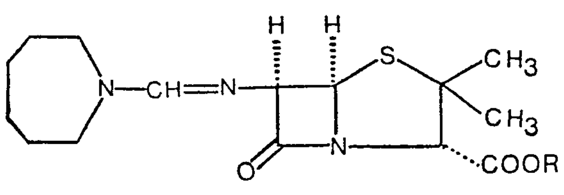

(I)

in der R Wasserstoff oder eine leicht hydrolysierbare Estergruppe darstellt,
sowie von pharmazeutisch verträglichen Salzen dieser Verbindungen, dadurch gekennzeichnet, dass man eine Verbindung der allgemeinen Formel

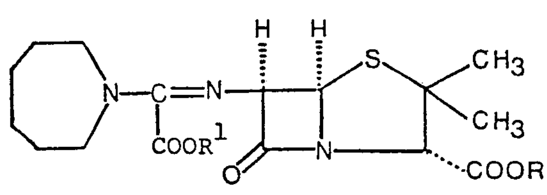

(II)

in der R die oben gegebene Bedeutung hat und $R^1$ eine hydrogenolytisch abspaltbare Gruppe bedeutet oder ein Salz einer solchen Verbindung in Gegenwart von Palladium/Kohle hydriert, wonach man erwünschtenfalls einen erhaltenen Ester der Formel I einer Esterspaltung unterwirft, erwünschtenfalls eine erhaltene Carbonsäure der Formel I mit einem die Estergruppe R abgebenden Mittel behandelt und erwünschtenfalls eine erhaltene Verbindung der Formel I in ein pharmazeutisch verträgliches Salz davon überführt.

2. Verfahren nach Anspruch 1, dadurch gekennzeichnet, dass $R^1$ eine Benzylgruppe bedeutet.

3. Verfahren nach Anspruch 1 oder 2, dadurch gekennzeichnet, dass man eine Ausgangsverbindung der Formel II einsetzt, worin R die Pivaloyloxymethylgruppe darstellt und Pivaloyloxymethyl 6-[(hexahydro-1H-azepin-1-yl)]-methylen-amino-penicillinat oder ein Salz davon isoliert.

4. 6-Aminopenicillansäurederivate der allgemeinen Formel

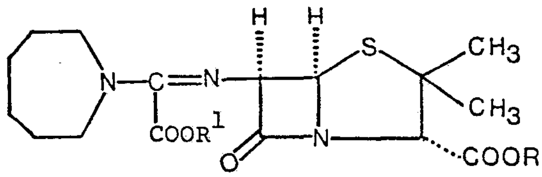

(II)

in der R Wasserstoff oder eine leicht hydrolysierbare Estergruppe und $R^1$ eine hydrogenolytisch abspaltbare Gruppe darstellt,
und Salze dieser Verbindungen.

5. 6-Aminopenicillansäurederivate gemäss Anspruch 4, dadurch gekennzeichnet, dass R Pivaloyloxymethyl darstellt.

6. 6-Aminopenicillansäurederivate gemäss Anspruch 4 oder 5, dadurch gekennzeichnet, dass $R^1$ Benzyl darstellt.

7. Methylen-(2S, 5R, 6R)-6-[[[(benzyloxy)carbonyl]-(hexahydro-1H-azepin-1-yl)methylen]amino]-7-oxo-4-thia-1-azabicyclo[3.2.0]heptan-2-carboxylatpivalat und Salze dieser Verbindung.

**Ansprüche** (für den Vertragsstaat AT)

1. Verfahren zur Herstellung von 6-Aminopenicillansäurederivaten der allgemeinen Formel

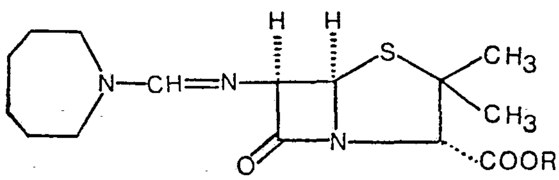

(I)

in der R Wasserstoff oder eine leicht hydrolysierbare Estergruppe darstellt,
sowie von pharmazeutisch verträglichen Salzen dieser Verbindungen, dadurch gekennzeichnet, dass man eine Verbindung der allgemeinen Formel

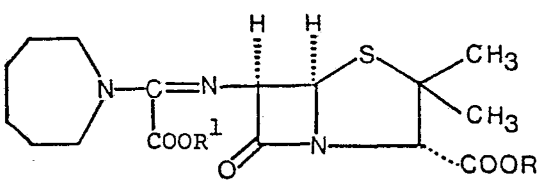

(II)

in der R die oben gegebene Bedeutung hat und $R^1$ eine hydrogenolytisch abspaltbare Gruppe bedeutet, oder ein Salz einer solchen Verbindung in Gegenwart von Palladium/Kohle hydriert, wonach man erwünschtenfalls einen erhaltenen Ester der Formel I einer Esterspaltung unterwirft, erwünschtenfalls eine erhaltene Carbonsäure der Formel I mit einem die Estergruppe R abgebenden Mittel behandelt und erwünschtenfalls eine erhaltene Verbindung der Formel I in ein pharmazeutisch verträgliches Salz davon überführt.

2. Verfahren nach Anspruch 1, dadurch gekennzeichnet, dass $R^1$ eine Benzylgruppe bedeutet.

3. Verfahren nach Anspruch 1 oder 2, dadurch gekennzeichnet, dass man eine Ausgangsverbindung der Formel II einsetzt, worin R die Pivaloyloxymethylgruppe darstellt und Pivaloyloxymethyl 6-[(hexahydro-1H-azepin-1-yl)]-methylenamino-penicillinat oder ein Salz davon isoliert.

**Claims** (for the Contracting States : CH, IT, LI, LU, NL, SE)

1. A process for the manufacture of 6-aminopenicillanic acid derivatives of the general formula

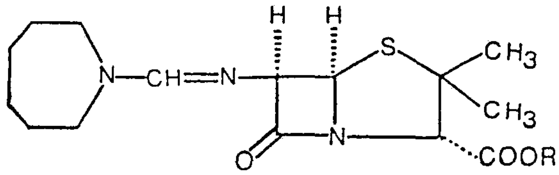

(I)

in which R represents hydrogen or a readily hydrolyzable ester group,
as well as of pharmaceutically compatible salts of these compounds, characterized by hydrogenating a compound of the general formula

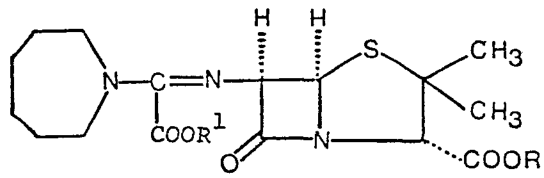

(II)

in which R has the significance given above and $R^1$ signifies a hydrogenolytically cleavable group, or a salt of such a compound in the presence of palladium/carbon, whereafter, if desired, an ester of formula I obtained is subjected to an ester cleavage, if desired a carboxylic acid of formula I obtained is treated with an agent yielding the ester group R and, if desired, a compound of formula I obtained is converted into a pharmaceutically compatible salt thereof.

2. A process according to claim 1, characterized in that $R^1$ signifies a benzyl group.

3. A process according to claim 1 or 2, characterized in that a starting compound of formula II wherein R represents the pivaloyloxymethyl group is used and pivaloyloxymethyl 6-[(hexahydro-1H-azepin-1-yl)]-methyleneamino-penicillanate or a salt thereof is isolated.

4. 6-Aminopenicillanic acid derivatives of the general formula

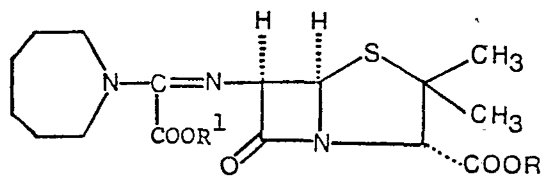

(II)

in which R represents hydrogen or a readily hydrolyzable ester group and $R^1$ represents a hydrogenolytically cleavable group,
and salts of these compounds.

5. 6-Aminopenicillanic acid derivatives in accordance with claim 4, characterized in that R represents pivaloyloxymethyl.

6. 6-Aminopenicillanic acid derivatives in accordance with claim 4 or 5, characterized in that $R^1$ represents benzyl.

7. Methylene-(2S, 5R, 6R)-6-[[[(benzyloxy)carbonyl]-(hexahydro-1H-azepin-1-yl)methylene] amino]-7-oxo-4-thia-1-azabicyclo[3.2.0]heptane-2-carboxylate pivalate and salts of this compound.

**Claims** (for the Contracting State AT)

1. A process for the manufacture of 6-aminopenicillanic acid derivatives of the general formula

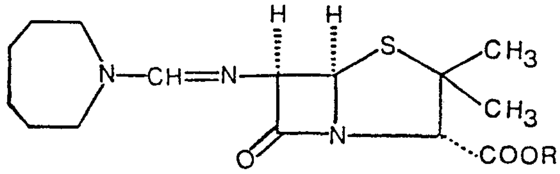

(I)

in which R represents hydrogen or a readily hydrolyzable ester group,
as well as of pharmaceutically compatible salts of these compounds, characterized by hydrogenating a compound of the general formula

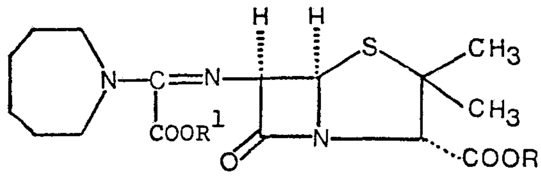

(II)

in which R has the significance given above and $R^1$ signifies a hydrogenolytically cleavable group, or a salt of such a compound in the presence of palladium/carbon, whereafter, if desired, an ester of formula I obtained is subjected to an ester cleavage, if desired a carboxylic acid of formula I obtained is treated with an agent yielding the ester group R and, if desired, a compound of formula I obtained is converted into a pharmaceutically compatible salt thereof.

2. A process according to claim 1, characterized in that $R^1$ signifies a benzyl group.

3. A process according to claim 1 or 2, characterized in that a starting compound of formula II wherein R represents the pivaloyloxymethyl group is used and pivaloyloxymethyl 6-[(hexahydro-1H-azepin-1-yl)]-methyleneamino-penicillanate or a salt thereof is isolated.

**Revendications** (pour les Etats contractants : CH, IT, LI, LU, NL, SE)

1. Procédé de préparation de dérivés d'acide 6-amino-pénicillanique de formule générale

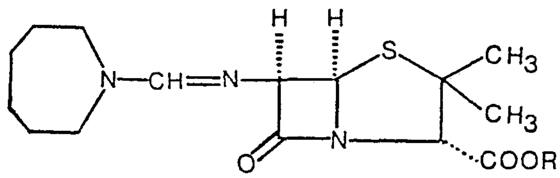

(I)

où R représente un hydrogène ou un groupe ester facilement hydrolysable, ainsi que des sels pharmaceutiquement acceptables de ces composés, caractérisé en ce qu'on hydrogène un composé de formule générale

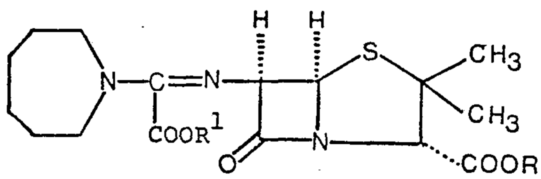

(II)

où R a la signification donnée ci-dessus et $R^1$ représente un groupe séparable par hydrogénolyse, ou un sel d'un tel composé, en présence de palladium/charbon, après quoi si on le désire on soumet un ester de formule I obtenu à une saponification, si on le désire on traite un acide carboxylique de formule I obtenu avec un agent donnant le groupe ester R et si on le désire on transforme un composé de formule I obtenu en un de ses sels pharmaceutiquement acceptable.

2. Procédé selon la revendication 1, caractérisé en ce que $R^1$ représente un groupe benzyle.

3. Procédé selon l'une des revendications 1 et 2, caractérisé en ce qu'on utilise un composé de départ de formule II, où R représente le groupe pivaloyloxyméthyle, et on isole le pivaloyloxyméthyl-6-[(hexahydro-1H-azépin-1-yl)]-méthylène-amino-pénicillinate ou un de ses sels.

4. Dérivés d'acide 6-aminopénicillanique de formule générale

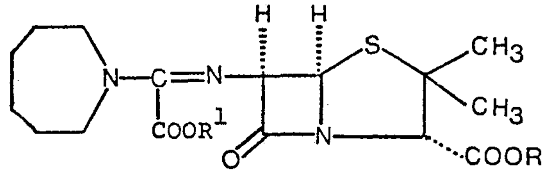

(II)

où R représente un hydrogène ou un groupe ester facilement hydrolysable et $R^1$ représente un groupe séparable par hydrogénolyse, et les sels de ces composés.

5. Dérivés d'acide 6-aminopénicillanique selon la revendication 4, caractérisés en ce que R représente un pivaloyloxyméthyle.

6. Dérivés d'acide 6-aminopénicillanique selon l'une des revendications 4 ou 5, caractérisés en ce que $R^1$ représente un benzyle.

7. Méthylène-(2S, 5R, 6R)-6-[[[(benzyloxy)carbonyl]-(hexahydro-1H-azépin-1-yl)méthylène]amino]-7-oxo-4-thia-1-azabicyclo[3.2.0]heptane-2-carboxylate-pivalate et les sels de ce composé.

**Revendications** (pour l'Etat contractant AT)

1. Procédé de préparation de dérivés d'acide 6-amino-pénicillanique de formule générale

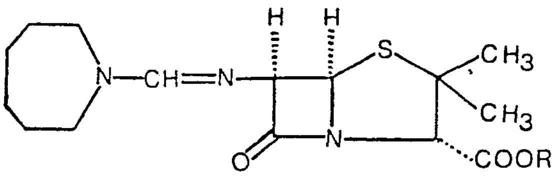

(I)

où R représente un hydrogène ou un groupe ester facilement hydrolysable, ainsi que des sels pharmaceutiquement acceptables de ces composés, caractérisé en ce qu'on hydrogène un composé de formule générale

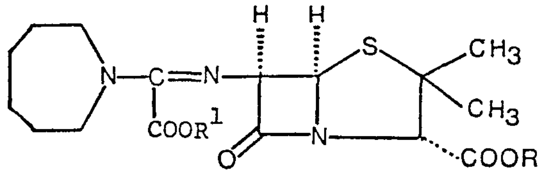

(II)

où R a la signification donnée ci-dessus et $R^1$ représente un groupe séparable par hydrogénolyse, ou un sel d'un tel composé en présence de palladium/charbon, après quoi si on le désire ou soumet un ester de formule I obtenu à une saponification, si on le désire on traite un acide carboxylique de formule I obtenu avec un agent donnant le groupe ester R et si on le désire on transforme un composé de formule I obtenu en un de ses sels pharmaceutiquement acceptables.

2. Procédé selon la revendication 1, caractérisé en ce que $R^1$ représente un groupe benzyle.

3. Procédé selon l'une des revendications 1 et 2, caractérisé en ce qu'on utilise un composé de départ de formule II où R représente le groupe pivaloyloxyméthyle et en ce qu'on isole le pivaloyloxyméthyl-6-[(hexahydro-1H-azépin-1-yl)]-méthylène-amino-pénicillinate ou un de ses sels.